# EUROPEAN PATENT APPLICATION

(11) **EP 4 691 402 A2**
(43) Date of publication of application: **11.02.2026**
(21) Application number: 25214051.2
(22) Date of filing: 25.10.2022
(51) Int. Cl.: A61B 18/26

(54) **OPTICAL EMITTER HOUSING ASSEMBLY FOR INTRAVASCULAR LITHOTRIPSY DEVICE**

(30) Priority: 14.12.2021 US 202163289294 P; 26.04.2022 US 202263335131 P; 20.10.2022 US 202217970363
(62) Divisional of application: 22812939.1
(71) Applicant: Bolt Medical, Inc., Carlsbad, CA 92008 (US)
(72) Inventor: SCHULTHEIS, Eric, San Clemente, CA (US); SALINAS, Alvin, San Marcos, 92069 (US); DUONG, Alan, San Diego, 92120 (US)
(74) Representative: Vossius & Partner Patentanwälte Rechtsanwälte mbB

(57) **Abstract**

The present invention relates to an emitter assembly for a catheter system for treating a treatment site within or adjacent to a blood vessel or a heart valve within a body of a patient, the emitter assembly comprising (i) at least a portion of an energy guide having a guide distal end that is selectively positioned near the treatment site, (ii) a plasma generator, and (iii) an emitter housing that is secured to each of the energy guide and the plasma generator to maintain a relative position between the guide distal end of the energy guide and the plasma generator, the energy guide being configured to receive energy from an energy source and direct the energy toward the plasma generator to generate a plasma bubble in a catheter fluid, and the plasma generator being configured to direct energy from the plasma bubble toward the treatment site, wherein the emitter housing includes a first housing that is secured to the energy guide at or near the guide distal end, and wherein the first housing section includes a guide aperture, at least a portion of the energy guide being secured within the guide aperture.

## Description

### RELATED APPLICATIONS

This application is related to U.S. Provisional Application Serial No. 63/289,294, filed on December 14, 2021, and entitled "OPTICAL EMITTER HOUSING ASSEMBLY FOR INTRAVASCULAR LITHOTRIPSY DEVICE", on U.S. Provisional Application Serial No. 63/335,131, filed on April 26, 2022, and entitled "OPTICAL EMITTER HOUSING ASSEMBLY FOR INTRAVASCULAR LITHOTRIPSY DEVICE", and on U.S. Patent Application Serial No. 17/970,363 filed on October 20, 2022, and entitled "OPTICAL EMITTER HOUSING ASSEMBLY FOR INTRAVASCULAR LITHOTRIPSY DEVICE". As far as permitted, the contents of U.S. Provisional Application Serial Nos. 63/289,294 and 63/335,131, and U.S. Patent Application Serial No. 17/970,363, are incorporated in their entirety herein by reference.

### BACKGROUND

Vascular lesions (also referred to herein as a "treatment site") within vessels in the body can be associated with an increased risk for major adverse events, such as myocardial infarction, embolism, deep vein thrombosis, stroke, and the like. Severe vascular lesions, such as severely calcified vascular lesions, can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion.

Intravascular lithotripsy is one method that has been recently used with some success for breaking up vascular lesions within vessels in the body. Intravascular lithotripsy utilizes a combination of pressure waves and bubble dynamics that are generated intravascularly in a fluid-filled balloon catheter. In particular, during an intravascular lithotripsy treatment, a high energy source is used to generate plasma and ultimately pressure waves as well as a rapid bubble expansion within a fluid-filled balloon to crack calcification at a treatment site within the vasculature that includes one or more vascular lesions. The associated rapid bubble formation from the plasma initiation and resulting localized fluid velocity within the balloon transfers mechanical energy through the incompressible fluid to impart a fracture force on the intravascular calcium, which is opposed to the balloon wall. The rapid change in fluid momentum upon hitting the balloon wall is known as hydraulic shock, or water hammer.

It is desired to more accurately and precisely direct and/or concentrate energy generated within the fluid-filled balloon so as to impart pressure onto and induce fractures at a treatment site within or adjacent to a blood vessel wall.

There is an ongoing desire to enhance vessel patency and optimization of therapy delivery parameters within an intravascular lithotripsy catheter system in a manner that is consistently manufacturable.

### SUMMARY

The present invention is directed toward a catheter system for placement within a blood vessel having a vessel wall. The catheter system can be used for treating a treatment site within or adjacent to the vessel wall or a heart valve within a body of a patient. In various embodiments, the catheter system includes an energy source, a catheter fluid, and an emitter assembly. The energy source generates energy. The emitter assembly includes (i) at least a portion of an energy guide having a guide distal end that is selectively positioned near the treatment site, (ii) a plasma generator, and (iii) an emitter housing that is secured to each of the energy guide and the plasma generator to maintain a relative position between the guide distal end of the energy guide and the plasma generator. The energy guide is configured to receive energy from the energy source and direct the energy toward the plasma generator to generate a plasma bubble in the catheter fluid. The plasma generator directs energy from the plasma bubble toward the treatment site.

In some embodiments, the emitter housing includes (i) a first housing section that is secured to the energy guide at or near the guide distal end, (ii) a second housing section that is one of secured to and integrally formed with the plasma generator, and (iii) a connector section that is coupled to and extends between the first housing section and the second housing section.

In certain embodiments, the first housing section is substantially cylindrical-shaped. In some such embodiments, the first housing section includes a small, housing gap that extends fully along a length of the first housing section and that allows for slight expansion or contraction of the first housing section due to changes in environmental conditions.

In certain embodiments, the first housing section includes a guide aperture; and at least a portion of the energy guide is secured within the guide aperture.

In some embodiments, the catheter system further includes adhesive material that is configured to secure the first housing section to the energy guide at or near the guide distal end.

In certain embodiments, the first housing section includes a first housing port through which the adhesive material can be provided between the first housing section and the energy guide so as to secure the first housing section to the energy guide at or near the guide distal end.

In some embodiments, the second housing section is substantially cylindrical-shaped.

In certain embodiments, the second housing section includes a small, housing gap that extends fully along a length of the second housing section and that allows for slight expansion or contraction of the second housing section due to changes in environmental conditions.

In certain embodiments, the second housing section includes a generator aperture; and at least a portion of the plasma generator is secured within the generator aperture.

In some embodiments, the catheter system further includes adhesive material that is configured to secure the second housing section to the plasma generator.

In certain embodiments, the second housing section includes a second housing port through which the adhesive material can be provided between the second housing section and the plasma generator so as to secure the second housing section to the plasma generator.

In other embodiments, the second housing section is integrally formed with the plasma generator.

In certain embodiments, the connector section includes a section opening; and the plasma generator directs the energy from the plasma bubble through the section opening and toward the treatment site.

In some embodiments, the connector section is partially cylindrical-shaped; and the section opening extends fully along a length of the connector section.

In some embodiments, the plasma generator has a proximal end that is angled so as to direct the energy from the plasma bubble through the section opening and toward the treatment site.

In certain embodiments, the proximal end of the plasma generator is angled at between approximately 5 degrees and 45 degrees relative to a flat, perpendicular configuration.

In some embodiments, the catheter system further includes a reinforcement cover that is positioned to substantially encircle the emitter housing.

In one embodiment, the reinforcement cover includes a polyimide tube.

In certain embodiments, the catheter system further includes a guidewire lumen that includes an outer surface having a groove; and the emitter housing is positioned within the groove formed along the outer surface of the guidewire lumen.

In some embodiments, the catheter system further includes a first assembly attacher that is positioned adjacent to the first housing section, and a second assembly attacher that is positioned adjacent to the second housing section, to hold the emitter housing within the groove formed along the outer surface of the guidewire lumen.

In some embodiments, the catheter system further includes a balloon including a balloon wall that defines a balloon interior, the balloon being configured to retain the catheter fluid within the balloon interior.

In various embodiments, the guide distal end, the plasma generator and the emitter housing are positioned within the balloon interior.

In certain such embodiments, the balloon is selectively inflatable with the catheter fluid to expand to an inflated state, and when the balloon is in the inflated state the balloon wall is configured to be positioned substantially adjacent to the treatment site.

In some embodiments, the plasma generator is configured to direct the energy from the plasma bubble toward a portion of the balloon wall that is positioned substantially adjacent to the treatment site.

In certain embodiments, the energy guide generates one or more pressure waves in the catheter fluid that impart a force upon the treatment site.

In some embodiments, the energy guide includes an optical fiber.

In various embodiments, the energy source includes a laser.

In certain embodiments, the catheter fluid includes one of a wetting agent and a surfactant.

The present invention is further directed toward a method for treating a treatment site within or adjacent to a blood vessel within a body of a patient, the method including the steps of: generating energy with an energy source; positioning an emitter assembly within a catheter fluid near the treatment site, the emitter assembly including (i) at least a portion of an energy guide having a guide distal end that is selectively positioned near the treatment site, (ii) a plasma generator, and (iii) an emitter housing that is secured to each of the energy guide and the plasma generator to maintain a relative position between the guide distal end of the energy guide and the plasma generator; receiving energy from the energy source with the energy guide; generating a plasma bubble in the catheter fluid with the energy from the energy guide that is directed toward the plasma generator; and directing energy from the plasma bubble with the plasma generator toward the treatment site.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a simplified schematic cross-sectional view illustration of an embodiment of a catheter system in accordance with various embodiments, the catheter system including an emitter assembly that includes at least a portion of an energy guide;
Figure 2 is a simplified schematic cross-sectional view illustration of a portion of an embodiment of the catheter system including an embodiment of the emitter assembly;
Figure 3 is a simplified schematic perspective view illustration of the emitter assembly illustrated in Figure 2;
Figure 4 is a simplified schematic exploded view illustration of the emitter assembly illustrated in Figure 2;
Figure 5 is a simplified schematic perspective view illustration of the emitter assembly illustrated in Figure 2 that is secured to a guidewire lumen of the catheter system;
Figure 6 is a simplified schematic perspective view illustration of another embodiment of the emitter assembly;
Figure 7 is a simplified schematic end view illustration of a portion of the emitter assembly illustrated in Figure 6; and
Figure 8 is a simplified schematic perspective view illustration of still another embodiment of the emitter assembly.

While embodiments of the present invention are susceptible to various modifications and alternative forms, specifics thereof have been shown by way of example and drawings, and are described in detail herein. It is understood, however, that the scope herein is not limited to the particular embodiments described. On the contrary, the intention is to cover modifications, equivalents, and alternatives falling within the spirit and scope herein.

### DESCRIPTION

Treatment of vascular lesions can reduce major adverse events or death in affected subjects. As referred to herein, a major adverse event is one that can occur anywhere within the body due to the presence of a vascular lesion. Major adverse events can include, but are not limited to, major adverse cardiac events, major adverse events in the peripheral or central vasculature, major adverse events in the brain, major adverse events in the musculature, or major adverse events in any of the internal organs.

As used herein, the terms "treatment site, "intravascular lesion" and "vascular lesion" are used interchangeably unless otherwise noted. As such, the intravascular lesions and/or the vascular lesions are sometimes referred to herein simply as "lesions".

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention as illustrated in the accompanying drawings. The same or similar nomenclature and/or reference indicators will be used throughout the drawings and the following detailed description to refer to the same or like parts.

In the interest of clarity, not all of the routine features of the implementations described herein are shown and described. It is appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is recognized that such a development effort might be complex and time-consuming, but would nevertheless be a routine undertaking of engineering for those of ordinary skill in the art having the benefit of this disclosure.

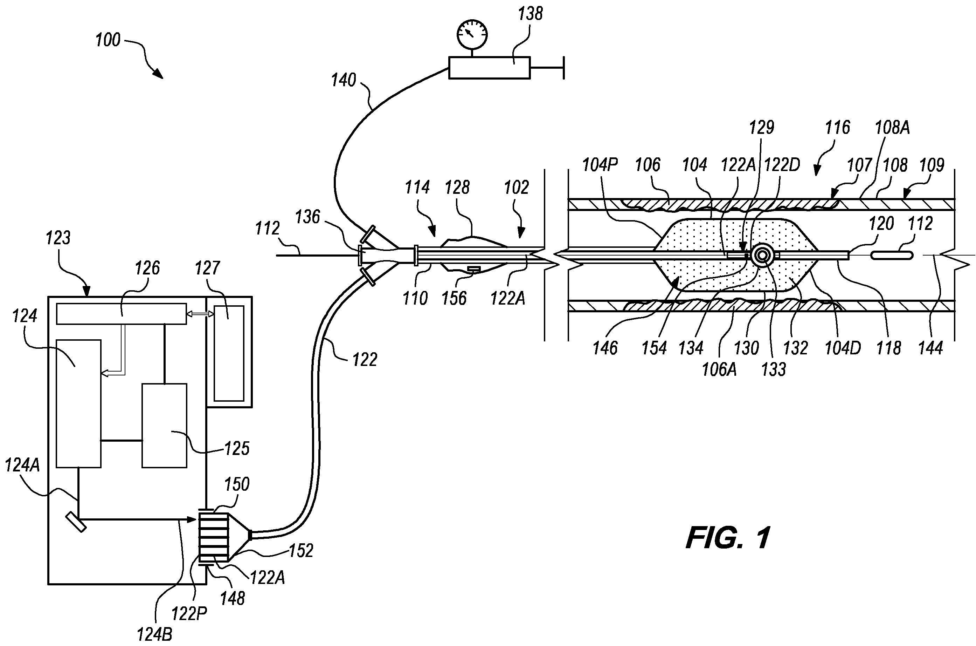

The catheter systems disclosed herein can include many different forms. Referring now to Figure 1, a simplified schematic cross-sectional view illustration is shown of a catheter system 100 in accordance with various embodiments. The catheter system 100 is suitable for imparting pressure waves to induce fractures in one or more treatment sites within or adjacent a vessel wall of a blood vessel or adjacent to a heart valve within a body of a patient. In the embodiment illustrated in Figure 1, the catheter system 100 can include one or more of a catheter 102, an energy guide bundle 122 including one or more energy guides 122A, a source manifold 136, a fluid pump 138, a system console 123 including one or more of an energy source 124, a power source 125, a system controller 126, and a graphic user interface 127 (a "GUI"), a handle assembly 128, and an emitter assembly 129. In various embodiments, the emitter assembly 129 includes and/or incorporates at least a portion of the energy guides 122A, and the emitter assembly 129 is configured to direct and/or concentrate energy toward one or more treatment sites 106A at a treatment site 106 within or adjacent to a vessel wall 108A of a blood vessel 108 or a heart valve within a body 107 of a patient 109. Alternatively, the catheter system 100 can include more components or fewer components than those specifically illustrated and described in relation to Figure 1.

The catheter 102 is configured to move to the treatment site 106 within or adjacent to the vessel wall 108A of the blood vessel 108 or a heart valve within the body 107 of the patient 109. The treatment site 106 can include one or more vascular lesions 106A such as calcified vascular lesions, for example. Additionally, or in the alternative, the treatment site 106 can include vascular lesions 106A such as fibrous vascular lesions. Still alternatively, in some implementations, the catheter 102 can be used at a treatment site 106 within or adjacent to a heart valve within the body 107 of the patient 109.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter 102 and/or the catheter shaft 110 can also include a guidewire lumen 118 which is configured to move over the guidewire 112. As utilized herein, the guidewire lumen 118 defines a conduit through which the guidewire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a catheter fluid 132 to expand from a deflated state suitable for advancing the catheter 102 through a patient's vasculature, to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment site 106. It is appreciated that although Figure 1 illustrates the balloon wall 130 of the balloon 104 being shown spaced apart from the treatment site 106 of the blood vessel 108 or a heart valve when in the inflated state, this is done for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloons 104 are made from silicone. In other embodiments, the balloon 104 can be made from materials such as polydimethylsiloxane (PDMS), polyurethane, polymers such as PEBAX^{™} material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloon 104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106, and, thus, may be usable for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

The balloon 104 can be inflated to inflation pressures of between approximately one atmosphere (atm) and 70 atm. In some embodiments, the balloon 104 can be inflated to inflation pressures of from at least 20 atm to 60 atm. In other embodiments, the balloon 104 can be inflated to inflation pressures of from at least six atm to 20 atm. In still other embodiments, the balloon 104 can be inflated to inflation pressures of from at least three atm to 20 atm. In yet other embodiments, the balloon 104 can be inflated to inflation pressures of from at least two atm to ten atm.

The balloon 104 can have various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloon 104 can include a drug eluting coating or a drug eluting stent structure. The drug eluting coating or drug eluting stent can include one or more therapeutic agents including anti-inflammatory agents, anti-neoplastic agents, anti-angiogenic agents, and the like.

The catheter fluid 132 can be a liquid or a gas. Some examples of the catheter fluid 132 suitable for use can include, but are not limited to one or more of water, saline, contrast medium, fluorocarbons, perfluorocarbons, gases, such as carbon dioxide, or any other suitable catheter fluid 132. In some embodiments, the catheter fluid 132 can be used as a base inflation fluid. In some embodiments, the catheter fluid 132 can include a mixture of saline to contrast medium in a volume ratio of approximately 50:50. In other embodiments, the catheter fluid 132 can include a mixture of saline to contrast medium in a volume ratio of approximately 25:75. In still other embodiments, the catheter fluid 132 can include a mixture of saline to contrast medium in a volume ratio of approximately 75:25. However, it is understood that any suitable ratio of saline to contrast medium can be used. The catheter fluid 132 can be tailored on the basis of composition, viscosity, and the like so that the rate of travel of the pressure waves are appropriately manipulated. In certain embodiments, the catheter fluids 132 suitable for use are biocompatible. A volume of catheter fluid 132 can be tailored by the chosen energy source 124 and the type of catheter fluid 132 used.

In certain embodiments, the catheter fluid 132 can include a wetting agent or surface-active agent (surfactant). These compounds can lower the tension between solid and liquid matter. These compounds can act as emulsifiers, dispersants, detergents, and water infiltration agents. Wetting agents or surfactants reduce surface tension of the liquid and allow it to fully wet and come into contact with optical components (such as the energy guide(s) 122A) and mechanical components (such as other portions of the emitter assembly(s) 129). This reduces or eliminates the accumulation of bubbles and pockets or inclusions of gas within the emitter assembly 129. Nonexclusive examples of chemicals that can be used as wetting agents include, but are not limited to, Benzalkonium Chloride, Benzethonium Chloride, Cetylpyridinium Chloride, Poloxamer 188, Poloxamer 407, Polysorbate 20, Polysorbate 40, and the like. Non-exclusive examples of surfactants can include, but are not limited to, ionic and non-ionic detergents, and Sodium stearate. Another suitable surfactant is 4-(5-dodecyl) benzenesulfonate. Other examples can include docusate (dioctyl sodium sulfosuccinate), alkyl ether phosphates, and perfluorooctanesulfonate (PFOS), to name a few.

By using a wetting agent or surfactant, direct liquid contact with the energy guide 122A allows the energy to be more efficiently converted into a plasma. Using the wetting agent or surfactant with the small dimensions of the optical and mechanical components used in the emitter assembly 129 and other parts of the catheter 102, it is less difficult to achieve greater (or complete) wetting. Decreasing the surface tension of the liquid can decrease the difficulty for such small structures to be effectively wetted by the liquid and therefore be nearly or completely immersed. By reducing or eliminating air or other gas bubbles from adhering to the optical and mechanical structure and energy guides 122A, considerable increase in efficiency of the device can occur.

The specific percentage of the wetting agent or surfactant can be varied to suit the design parameters of the catheter system 100 and/or the emitter assembly 129 being used. In one embodiment, the percentage of the wetting agent or surfactant can be less than approximately 50% by volume of the catheter fluid 132. In non-exclusive alternative embodiments, the percentage of the wetting agent or surfactant can be less than approximately 40%, 30%, 20%, 10%, 5%, 2%, 1%, 0.1% or 0.01% by volume of the catheter fluid 132. Still alternatively, the percentage of the wetting agent or surfactant can fall outside of the foregoing ranges.

In some embodiments, the contrast agents used in the contrast media can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine-based contrast agents can be used. Suitable non-iodine containing contrast agents can include gadolinium (III)-based contrast agents. Suitable fluorocarbon and perfluorocarbon agents can include, but are not to be limited to, agents such as the perfluorocarbon dodecafluoropentane (DDFP, C5F12).

The catheter fluids 132 can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 µm) of the electromagnetic spectrum. Suitable absorptive agents can include those with absorption maxima along the spectrum from at least ten nm to 2.5 µm. Alternatively, the catheter fluids 132 can include those that include absorptive agents that can selectively absorb light in the mid-infrared region (e.g., at least 2.5 µm to 15 µm), or the far-infrared region (e.g., at least 15 µm to one mm) of the electromagnetic spectrum. In various embodiments, the absorptive agent can be those that have an absorption maximum matched with the emission maximum of the laser used in the catheter system 100. By way of non-limiting examples, various lasers usable in the catheter system 100 can include neodymium:yttrium-aluminum-garnet (Nd:YAG - emission maximum = 1064 nm) lasers, holmium:YAG (Ho:YAG - emission maximum = 2.1 µm) lasers, or erbium:YAG (Er:YAG - emission maximum = 2.94 µm) lasers. In some embodiments, the absorptive agents can be water-soluble. In other embodiments, the absorptive agents are not water-soluble. In some embodiments, the absorptive agents used in the catheter fluids 132 can be tailored to match the peak emission of the energy source 124. Various energy sources 124 having emission wavelengths of at least ten nanometers to one millimeter are discussed elsewhere herein.

The catheter shaft 110 of the catheter 102 can be coupled to the one or more energy guides 122A of the energy guide bundle 122 that are in optical communication with the energy source 124. The energy guide(s) 122A can be disposed along the catheter shaft 110 and within the balloon 104. In some embodiments, each energy guide 122A can be an optical fiber and the energy source 124 can be a laser. The energy source 124 can be in optical communication with the energy guides 122A at the proximal portion 114 of the catheter system 100.

In some embodiments, the catheter shaft 110 can be coupled to multiple energy guides 122A such as a first energy guide, a second energy guide, a third energy guide, etc., which can be disposed at any suitable positions about and/or relative to the guidewire lumen 118 and/or the catheter shaft 110. For example, in certain non-exclusive embodiments, two energy guides 122A can be spaced apart by approximately 180 degrees about the circumference of the guidewire lumen 118 and/or the catheter shaft 110; three energy guides 122A can be spaced apart by approximately 120 degrees about the circumference of the guidewire lumen 118 and/or the catheter shaft 110; or four energy guides 122A can be spaced apart by approximately 90 degrees about the circumference of the guidewire lumen 118 and/or the catheter shaft 110. Still alternatively, multiple energy guides 122A need not be uniformly spaced apart from one another about the circumference of the guidewire lumen 118 and/or the catheter shaft 110. More particularly, it is further appreciated that the energy guides 122A can be disposed uniformly or non-uniformly about the guidewire lumen 118 and/or the catheter shaft 110 to achieve the desired effect in the desired locations.

In certain embodiments, the guidewire lumen 118 can have a grooved outer surface, with the grooves extending in a generally longitudinal direction along the guidewire lumen 118. In such embodiments, each of the energy guides 122A and/or the emitter assembly(s) 129 can be positioned, received and retained within an individual groove formed along and/or into the outer surface of the guidewire lumen 118. Alternatively, the guidewire lumen 118 can be formed without a grooved outer surface, and the position of the energy guides 122A and/or the emitter assembly(s) 129 relative to the guidewire lumen 118 can be maintained in another suitable manner.

The catheter system 100 and/or the energy guide bundle 122 can include any number of energy guides 122A in optical communication with the energy source 124 at the proximal portion 114, and with the catheter fluid 132 within the balloon interior 146 of the balloon 104 at the distal portion 116. For example, in some embodiments, the catheter system 100 and/or the energy guide bundle 122 can include from one energy guide 122A to greater than 30 energy guides 122A. Alternatively, in other embodiments, the catheter system 100 and/or the energy guide bundle 122 can include greater than 30 energy guides 122A.

The energy guides 122A can have any suitable design for purposes of generating plasma and/or pressure waves in the catheter fluid 132 within the balloon interior 146. Thus, the general description of the energy guides 122A as light guides is not intended to be limiting in any manner, except for as set forth in the claims appended hereto. More particularly, although the catheter systems 100 are often described with the energy source 124 as a light source and the one or more energy guides 122A as light guides, the catheter system 100 can alternatively include any suitable energy source 124 and energy guides 122A for purposes of generating the desired plasma in the catheter fluid 132 within the balloon interior 146. For example, in one non-exclusive alternative embodiment, the energy source 124 can be configured to provide high voltage pulses, and each energy guide 122A can include an electrode pair including spaced apart electrodes that extend into the balloon interior 146. In such embodiment, each pulse of high voltage is applied to the electrodes and forms an electrical arc across the electrodes, which, in turn, generates the plasma and forms the pressure waves in the catheter fluid 132 that are utilized to provide the fracture force onto the vascular lesions 106A at the treatment site 106. Still alternatively, the energy source 124 and/or the energy guides 122A can have another suitable design and/or configuration.

In certain embodiments, the energy guides 122A can include an optical fiber or flexible light pipe. The energy guides 122A can be thin and flexible and can allow light signals to be sent with very little loss of strength. The energy guides 122A can include a core surrounded by a cladding about its circumference. In some embodiments, the core can be a cylindrical core or a partially cylindrical core. The core and cladding of the energy guides 122A can be formed from one or more materials, including but not limited to one or more types of glass, silica, or one or more polymers. The energy guides 122A may also include a protective coating, such as a polymer. It is appreciated that the index of refraction of the core will be greater than the index of refraction of the cladding.

Each energy guide 122A can guide energy along its length from a guide proximal end 122P to the guide distal end 122D having at least one optical window (not shown) that is positioned within the balloon interior 146.

The energy guides 122A can assume many configurations about and/or relative to the catheter shaft 110 of the catheter 102. In some embodiments, the energy guides 122A can run parallel to the longitudinal axis 144 of the catheter shaft 110. In some embodiments, the energy guides 122A can be physically coupled to the catheter shaft 110. In other embodiments, the energy guides 122A can be disposed along a length of an outer diameter of the catheter shaft 110. In yet other embodiments, the energy guides 122A can be disposed within one or more energy guide lumens within the catheter shaft 110.

The energy guides 122A can also be disposed at any suitable positions about the circumference of the guidewire lumen 118 and/or the catheter shaft 110, and the guide distal end 122D of each of the energy guides 122A can be disposed at any suitable longitudinal position relative to the length of the balloon 104 and/or relative to the length of the guidewire lumen 118 to more effectively and precisely impart pressure waves for purposes of disrupting the vascular lesions 106A at the treatment site 106.

In certain embodiments, the energy guides 122A can include one or more photoacoustic transducers 154, where each photoacoustic transducer 154 can be in optical communication with the energy guide 122A within which it is disposed. In some embodiments, the photoacoustic transducers 154 can be in optical communication with the guide distal end 122D of the energy guide 122A. In such embodiments, the photoacoustic transducers 154 can have a shape that corresponds with and/or conforms to the guide distal end 122D of the energy guide 122A.

The photoacoustic transducer 154 is configured to convert light energy into an acoustic wave at or near the guide distal end 122D of the energy guide 122A. The direction of the acoustic wave can be tailored by changing an angle of the guide distal end 122D of the energy guide 122A.

In certain embodiments, the photoacoustic transducers 154 disposed at the guide distal end 122D of the energy guide 122A can assume the same shape as the guide distal end 122D of the energy guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The energy guide 122A can further include additional photoacoustic transducers 154 disposed along one or more side surfaces of the length of the energy guide 122A.

In some embodiments, the energy guides 122A and/or the emitter assembly 129 can further include one or more diverting features or "diverters" (not shown in Figure 1), such as within the energy guide 122A and/or near the guide distal end 122D of the energy guide 122A, that are configured to direct energy from the energy guide 122A toward a side surface which can be located at or near the guide distal end 122D of the energy guide 122A, before the energy is directed toward the balloon wall 130. A diverting feature can include any feature of the system that diverts energy from the energy guide 122A away from its axial path toward a side surface of the energy guide 122A. The energy guides 122A can each include one or more optical windows disposed along the longitudinal or circumferential surfaces of each energy guide 122A and in optical communication with a diverting feature. Stated in another manner, the diverting features can be configured to direct energy in the energy guide 122A toward a side surface that is at or near the guide distal end 122D, where the side surface is in optical communication with an optical window. The optical windows can include a portion of the energy guide 122A that allows energy to exit the energy guide 122A from within the energy guide 122A, such as a portion of the energy guide 122A lacking a cladding material on or about the energy guide 122A.

Examples of the diverting features suitable for use include a reflecting element, a refracting element, and a fiber diffuser. The diverting features suitable for focusing energy away from the tip of the energy guides 122A can include, but are not to be limited to, those having a convex surface, a gradient-index (GRIN) lens, and a mirror focus lens. Upon contact with the diverting feature, the energy is diverted within the energy guide 122A to one or more of a plasma generator 133 and the photoacoustic transducer 154 that is in optical communication with a side surface of the energy guide 122A. When utilized, the photoacoustic transducer 154 then converts light energy into an acoustic wave that extends away from the side surface of the energy guide 122A.

Additionally, or in the alternative, in certain embodiments, such diverting features that can be incorporated into the energy guides 122A, can also be incorporated into the design of the emitter assembly 129 and/or the plasma generator 133 for purposes of directing and/or concentrating acoustic and mechanical energy toward specific areas of the balloon wall 130 in contact with the vascular lesions 106A at the treatment site 106 to impart pressure onto and induce fractures in such vascular lesions 106A.

The source manifold 136 can be positioned at or near the proximal portion 114 of the catheter system 100. The source manifold 136 can include one or more proximal end openings that can receive the one or more energy guides 122A of the energy guide bundle 122, the guidewire 112, and/or an inflation conduit 140 that is coupled in fluid communication with the fluid pump 138. The catheter system 100 can also include the fluid pump 138 that is configured to inflate the balloon 104 with the catheter fluid 132 as needed.

As noted above, in the embodiment illustrated in Figure 1, the system console 123 includes one or more of the energy source 124, the power source 125, the system controller 126, and the GUI 127. Alternatively, the system console 123 can include more components or fewer components than those specifically illustrated in Figure 1. For example, in certain non-exclusive alternative embodiments, the system console 123 can be designed without the GUI 127. Still alternatively, one or more of the energy source 124, the power source 125, the system controller 126, and the GUI 127 can be provided within the catheter system 100 without the specific need for the system console 123.

As shown, the system console 123, and the components included therewith, is operatively coupled to the catheter 102, the energy guide bundle 122, and the remainder of the catheter system 100. For example, in some embodiments, as illustrated in Figure 1, the system console 123 can include a console connection aperture 148 (also sometimes referred to generally as a "socket") by which the energy guide bundle 122 is mechanically coupled to the system console 123. In such embodiments, the energy guide bundle 122 can include a guide coupling housing 150 (also sometimes referred to generally as a "ferrule") that houses a portion, such as the guide proximal end 122P, of each of the energy guides 122A. The guide coupling housing 150 is configured to fit and be selectively retained within the console connection aperture 148 to provide the mechanical coupling between the energy guide bundle 122 and the system console 123.

The energy guide bundle 122 can also include a guide bundler 152 (or "shell") that brings each of the individual energy guides 122A closer together so that the energy guides 122A and/or the energy guide bundle 122 can be in a more compact form as it extends with the catheter 102 into the blood vessel 108 or a heart valve during use of the catheter system 100.

The energy source 124 can be selectively and/or alternatively coupled in optical communication with each of the energy guides 122A, such as to the guide proximal end 122P of each of the energy guides 122A, in the energy guide bundle 122. In particular, the energy source 124 is configured to generate energy in the form of a source beam 124A, such as a pulsed source beam, that can be selectively and/or alternatively directed to and received by each of the energy guides 122A in the energy guide bundle 122 as an individual guide beam 124B. Alternatively, the catheter system 100 can include more than one energy source 124. For example, in one non-exclusive alternative embodiment, the catheter system 100 can include a separate energy source 124 for each of the energy guides 122A in the energy guide bundle 122.

The energy source 124 can have any suitable design. In certain embodiments, the energy source 124 can be configured to provide sub-millisecond pulses of energy from the energy source 124 that are focused onto a small spot in order to couple it into the guide proximal end 122P of the energy guide 122A. Such pulses of energy are then directed and/or guided along the energy guides 122A to a location within the balloon interior 146 of the balloon 104, thereby inducing plasma formation in the catheter fluid 132 within the balloon interior 146 of the balloon 104, such as via the plasma generator 133 that can be located at or near the guide distal end 122D of the energy guide 122A. In particular, in such embodiments, the energy emitted at the guide distal end 122D of the energy guide 122A is directed toward and energizes the plasma generator 133 to form the plasma in the catheter fluid 132 within the balloon interior 146. The plasma formation causes rapid bubble formation, and imparts pressure waves upon the treatment site 106. An exemplary plasma-induced bubble 134 is illustrated in Figure 1.

In various non-exclusive alternative embodiments, the sub-millisecond pulses of energy from the energy source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of energy can be delivered to the treatment site 106 at a frequency that can be greater than 5000 Hz or less than one Hz, or any other suitable range of frequencies.

It is appreciated that although the energy source 124 is typically utilized to provide pulses of energy, the energy source 124 can still be described as providing a single source beam 124A, i.e. a single pulsed source beam.

The energy sources 124 suitable for use can include various types of light sources including lasers and lamps. Alternatively, the energy sources 124 can include any suitable type of energy source.

Suitable lasers can include short pulse lasers on the sub-millisecond timescale. In some embodiments, the energy source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths and energy levels that can be employed to achieve plasma in the catheter fluid 132 of the catheter 102. In various non-exclusive alternative embodiments, the pulse widths can include those falling within a range including from at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the energy sources 124 suitable for use in the catheter systems 100 can include those capable of producing light at wavelengths of from at least 750 nm to 2000 nm. In other embodiments, the energy sources 124 can include those capable of producing light at wavelengths of from at least 700 nm to 3000 nm. In still other embodiments, the energy sources 124 can include those capable of producing light at wavelengths of from at least 100 nm to ten micrometers (µm). Nanosecond lasers can include those having repetition rates of up to 200 kHz.

In some embodiments, the laser can include a Q-switched thulium:yttrium-aluminum-garnet (Tm:YAG) laser. In other embodiments, the laser can include a neodymium:yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers.

In still other embodiments, the energy source 124 can include a plurality of lasers that are grouped together in series. In yet other embodiments, the energy source 124 can include one or more low energy lasers that are fed into a high energy amplifier, such as a master oscillator power amplifier (MOPA). In still yet other embodiments, the energy source 124 can include a plurality of lasers that can be combined in parallel or in series to provide the energy needed to create the plasma bubble 134 in the catheter fluid 132.

The catheter system 100 can generate pressure waves having maximum pressures in the range of at least one megapascal (MPa) to 100 MPa. The maximum pressure generated by a particular catheter system 100 will depend on the energy source 124, the absorbing material, the bubble expansion, the propagation medium, the balloon material, and other factors. In various non-exclusive alternative embodiments, the catheter systems 100 can generate pressure waves having maximum pressures in the range of at least approximately two MPa to 50 MPa, at least approximately two MPa to 30 MPa, or approximately at least 15 MPa to 25 MPa.

The pressure waves can be imparted upon the treatment site 106 from a distance within a range from at least approximately 0.1 millimeters (mm) to greater than approximately 25 mm extending radially from the energy guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the energy guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide necessary power to each of the energy source 124, the system controller 126, the GUI 127, and the handle assembly 128. The power source 125 can have any suitable design for such purposes.

The system controller 126 is electrically coupled to and receives power from the power source 125. The system controller 126 is coupled to and is configured to control operation of each of the energy source 124 and the GUI 127. The system controller 126 can include one or more processors or circuits for purposes of controlling the operation of at least the energy source 124 and the GUI 127. For example, the system controller 126 can control the energy source 124 for generating pulses of energy as desired and/or at any desired firing rate.

The system controller 126 can also be configured to control operation of other components of the catheter system 100 such as the positioning of the catheter 102 adjacent to the treatment site 106, the inflation of the balloon 104 with the catheter fluid 132, etc. Further, or in the alternative, the catheter system 100 can include one or more additional controllers that can be positioned in any suitable manner for purposes of controlling the various operations of the catheter system 100. For example, in certain embodiments, an additional controller and/or a portion of the system controller 126 can be positioned and/or incorporated within the handle assembly 128.

The GUI 127 is accessible by the user or operator of the catheter system 100. The GUI 127 is electrically connected to the system controller 126. With such design, the GUI 127 can be used by the user or operator to ensure that the catheter system 100 is effectively utilized to impart pressure onto and induce fractures into the vascular lesions 106A at the treatment site 106. The GUI 127 can provide the user or operator with information that can be used before, during and after use of the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during use of the catheter system 100. In various embodiments, the GUI 127 can include one or more colors, different sizes, varying brightness, etc., that may act as alerts to the user or operator. Additionally, or in the alternative, the GUI 127 can provide audio data or information to the user or operator. The specifics of the GUI 127 can vary depending upon the design requirements of the catheter system 100, or the specific needs, specifications and/or desires of the user or operator.

As shown in Figure 1, the handle assembly 128 can be positioned at or near the proximal portion 114 of the catheter system 100, and/or near the source manifold 136. In this embodiment, the handle assembly 128 is coupled to the balloon 104 and is positioned spaced apart from the balloon 104. Alternatively, the handle assembly 128 can be positioned at another suitable location.

The handle assembly 128 is handled and used by the user or operator to operate, position and control the catheter 102. The design and specific features of the handle assembly 128 can vary to suit the design requirements of the catheter system 100. In the embodiment illustrated in Figure 1, the handle assembly 128 is separate from, but in electrical and/or fluid communication with one or more of the system controller 126, the energy source 124, the fluid pump 138, and the GUI 127. In some embodiments, the handle assembly 128 can integrate and/or include at least a portion of the system controller 126 within an interior of the handle assembly 128. For example, as shown, in certain such embodiments, the handle assembly 128 can include circuitry 156 that can form at least a portion of the system controller 126. In one embodiment, the circuitry 156 can include a printed circuit board having one or more integrated circuits, or any other suitable circuitry. In an alternative embodiment, the circuitry 156 can be omitted, or can be included within the system controller 126, which in various embodiments can be positioned outside of the handle assembly 128, such as within the system console 123. It is understood that the handle assembly 128 can include fewer or additional components than those specifically illustrated and described herein.

In various implementations, the emitter assembly 129 is configured to maintain a desired positioning between the guide distal end 122D of the energy guide 122A and the plasma generator 133, and to direct and/or concentrate energy generated in the catheter fluid 132 within the balloon interior 146 so as to impart pressure onto and induce fractures in vascular lesions 106A at the treatment site 106 within or adjacent to a vessel wall 108A of a blood vessel 108 or a heart valve. More particularly, by effectively maintaining the desired positioning between the guide distal end 122D of the energy guide 122A and the plasma generator 133, and with the particular design features that may be incorporated into the emitter assembly 129, the emitter assembly 129 is configured to concentrate and direct acoustic and/or mechanical energy toward specific areas of the balloon wall 130 in contact with the vascular lesions 106A at the treatment site 106 to enhance the delivery of such energy to the treatment site 106. Thus, the emitter assembly 129 is able to effectively improve the efficacy of the catheter system 100.

It is appreciated that, in some embodiments, a separate emitter assembly 129 can be included with and/or incorporated into each individual energy guide 122A. Alternatively, in other embodiments, a single emitter assembly 129 can be configured to operate in conjunction with more than one energy guide 122A. Still alternatively, each energy guide 122A need not have an emitter assembly 129 incorporated therein or associated therewith.

The design of the emitter assembly 129 and/or the specific positioning of the emitter assembly 129 can be varied to suit the requirements of the catheter system 100. In various embodiments, the emitter assembly 129 can utilize and/or incorporate at least a portion of the energy guide 122A, such as a portion that includes the guide distal end 122D of the energy guide 122A.

Various alternative embodiments of the emitter assembly 129 are illustrated and described in detail herein below within subsequent Figures.

As with all embodiments illustrated and described herein, various structures may be omitted from the figures for clarity and ease of understanding. Further, the figures may include certain structures that can be omitted without deviating from the intent and scope of the invention.

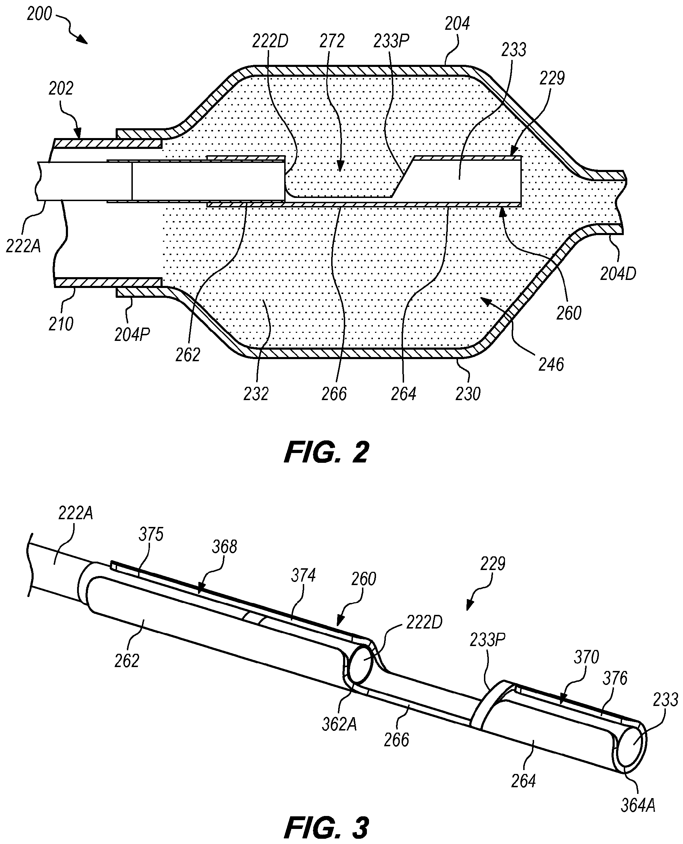

Figure 2 is a simplified schematic cross-sectional view illustration of a portion of an embodiment of the catheter system 200, including an embodiment of the emitter assembly 229. The design of the catheter system 200 can be varied. In various embodiments, as illustrated in Figure 2, the catheter system 200 can include a catheter 202 including a catheter shaft 210; a balloon 204 having a balloon wall 230 that defines a balloon interior 246, a balloon proximal end 204P, and a balloon distal end 204D; and a catheter fluid 232 that is retained substantially within the balloon interior 246; and the emitter assembly 229, which in certain embodiments can incorporate at least a portion of an energy guide 222A. Alternatively, in other embodiments, the catheter system 200 can include more components or fewer components than what is specifically illustrated and described herein. For example, certain components that were illustrated in Figure 1, such as the guidewire 112, the guidewire lumen 118, the source manifold 136, the fluid pump 138, the energy source 124, the power source 125, the system controller 126, the GUI 127, and the handle assembly 128, are not specifically illustrated in Figure 2 for purposes of clarity, but would likely be included in any embodiment of the catheter system 200.

The design and function of the catheter shaft 210, the balloon 204, and the catheter fluid 232 are substantially similar to what was illustrated and described herein above. Accordingly, a detailed description of such components will not be repeated.

The balloon 204 is again selectively movable between a deflated state suitable for advancing the catheter 202 through a patient's vasculature, and an inflated state suitable for anchoring the catheter 202 in position relative to the treatment site 106 (illustrated in Figure 1). In some embodiments, the balloon proximal end 204P can be coupled to the catheter shaft 210, and the balloon distal end 204D can be coupled to the guidewire lumen 118 (illustrated in Figure 1). The balloon 204 can again be inflated with the catheter fluid 232, such as from the fluid pump 138 (illustrated in Figure 1), that is directed into the balloon interior 246 of the balloon 204 via the inflation conduit 140 (illustrated in Figure 1).

Similar to previous embodiments, the energy guide 222A can include one or more photoacoustic transducers 154 (illustrated in Figure 1), where each photoacoustic transducer 154 can be in optical communication with the energy guide 222A within which it is disposed. In some embodiments, the photoacoustic transducers 154 can be in optical communication with the guide distal end 222D of the energy guide 222A. Alternatively, in other embodiments, the energy guide 222A can be designed without the one or more photoacoustic transducers 154.

In various embodiments, the emitter assembly 229 is configured to direct and/or concentrate energy generated in the catheter fluid 232 within the balloon interior 246 to impart pressure onto and induce fractures in vascular lesions 106A (illustrated in Figure 1) at the treatment site 106. More particularly, the emitter assembly 229 is configured to direct and concentrate acoustic and/or mechanical energy toward specific areas of the balloon wall 230 that are in contact with the vascular lesions 106A at the treatment site 106 to enhance the delivery of such energy to the treatment site 106. As illustrated in this embodiment, at least some of the components of the emitter assembly 229 are positioned within the balloon interior 246
The design of the emitter assembly 229 can be varied. As shown in Figure 2, in certain embodiments, the emitter assembly 229 includes at least a portion of the energy guide 222A, a plasma generator 233, and an emitter housing 260 that is coupled to and/or secured to the energy guide 222A and the plasma generator 233.

In some embodiments, as illustrated, the emitter housing 260 can include one or more of (i) a first housing section 262 that is coupled and/or secured to the energy guide 222A, such as at or near the guide distal end 222D of the energy guide 222A, (ii) a second housing section 264 that is coupled and/or secured to the plasma generator 233, and (iii) a connector section 266 that is coupled to, integrally formed with, and/or extends between the first housing section 262 and the second housing section 264. In such embodiments, the emitter housing 260 can be formed as a unitary structure that includes each of the first housing section 262, the second housing section 264 and the connector section 266; or the first housing section 262, the second housing section 264 and the connector section 266 of the emitter housing 260 can be formed as separate components that are secured to one another. Alternatively, the emitter housing 260 can include more components or fewer components than what is specifically illustrated in Figure 2.

As shown, the first housing section 262 of the emitter housing 260 is configured to be secured to and substantially encircle at least a portion of the energy guide 222A, such as at or near the guide distal end 222D of the energy guide 222A. In one such embodiment, the first housing section 262 of the emitter housing 260 is substantially annular-shaped and/or cylindrical-shaped, and includes a guide aperture 362A (illustrated in Figure 3) through and/or into which the energy guide 222A can be positioned. Alternatively, the first housing section 262 can have another suitable shape. As utilized herein, the description of the first housing section 262 as substantially encircling at least a portion of the energy guide 222A and/or being substantially annular-shaped and/or cylindrical-shaped is intended to signify that the first housing section 262 encircles at least approximately 90% to 95% of such portion of the energy guide 222A, but can further include a small housing gap 368 (illustrated in Figure 3) that extends fully along a length of the first housing section 262 and that allows for slight expansion or contraction of the first housing section 262 due to changes in environmental conditions in which the catheter system 200 is being used. The housing gap 368 allows for such potential expansion or contraction of the first housing section 262 without adversely impacting the structure of the guide distal end 222D of the energy guide 222A about which the first housing section 262 is positioned.

The first housing section 262 can be secured to a portion of the energy guide 222A, such as at or near the guide distal end 222D, in any suitable manner. For example, the first housing section 262 can be secured to a portion of the energy guide 222A with any suitable type of adhesive material. Alternatively, the first housing section 262 can be secured to a portion of the energy guide 222A in another suitable manner.

Somewhat similarly, as shown, the second housing section 264 of the emitter housing 260 is configured to be secured to and substantially encircle the plasma generator 233. In one such embodiment, the second housing section 264 of the emitter housing 260 is substantially annular-shaped and/or cylindrical-shaped, and includes a generator aperture 364A (illustrated in Figure 3) through and/or into which the plasma generator 233 can be positioned. Alternatively, the second housing section 264 can have another suitable shape. As utilized herein, the description of the second housing section 264 as substantially encircling the plasma generator 233 and/or being substantially annular-shaped and/or cylindrical-shaped is intended to signify that the second housing section 264 encircles at least approximately 90% to 95% of the plasma generator 233, but can further include a small housing gap 370 (illustrated in Figure 3) that extends fully along a length of the second housing section 264 and that allows for slight expansion or contraction of the second housing section 264 due to changes in environmental conditions in which the catheter system 200 is being used. The housing gap 370 allows for such potential expansion or contraction of the second housing section 264 without adversely impacting the structure of the plasma generator 233 about which the second housing section 264 is positioned.

The second housing section 264 can be secured to the plasma generator 233 in any suitable manner. For example, the second housing section 264 can be secured to the plasma generator 233 with any suitable type of adhesive material. Alternatively, the second housing section 264 can be secured to the plasma generator 233 in another suitable manner.

The connector section 266 of the emitter housing 260, as noted, is coupled to, integrally formed with, and/or extends between the first housing section 262 and the second housing section 264. In some embodiments, the connector section 266 can be partially annular-shaped and/or cylindrical-shaped, with a section opening 272 that extends fully along a length of the connector section 266 to help define the less than complete annular and/or cylindrical shape of the connector section 266, and that is configured such that the plasma energy formed in the catheter fluid 232 within the balloon interior 246 is directed and/or concentrated through the section opening 272 and toward the vascular lesions 106A formed at the treatment site 106. The size and orientation of the section opening 272 can be varied depending on the size and position of the vascular lesions 106A being treated with the catheter system 200. In some non-exclusive alternative embodiments, the section opening 272 can be less than approximately 10%, 15%, 20%, 25%, 30%, 35%, 40%, 45%, 50%, 55%, 60%, 65%, 70%, 75%, 80%, 85% or 90% of what would otherwise form a complete annular and/or cylindrical shape for the connector section 266. Stated in another manner, the connector section 266 can be formed as at least approximately 90%, 85%, 80%, 75%, 70%, 65%, 60%, 55%, 50%, 45%, 40%, 35%, 30%, 25%, 20%, 15% or 10% of a complete annular-shaped and/or cylinder-shaped body.

In various embodiments, the emitter housing 260 can be formed from long, narrow tubing (a hypotube) and from any suitable materials. For example, in certain non-exclusive embodiments, the emitter housing 260 can be formed from a hypotube including one or more metals such as titanium, stainless steel, tungsten, etc. Alternatively, the emitter housing 260 may be formed from a hypotube including plastics such as polyimide and nylon. Still alternatively, the emitter housing 260 may be injection molded and over molding processing can be used to secure either the energy guide 222A and/or the plasma generator 233 into place. Yet alternatively, the emitter housing 260 can be formed in another suitable manner and/or from other suitable materials. For example, in certain alternative embodiments, the features on the emitter housing 260 can be done by laser cutting, milling or swiss screw machining processes of a raw hypotube.

With such design of the emitter housing 260, a desired relative positioning can be effectively maintained between the guide distal end 222D of the energy guide 222A and the plasma generator 233. During use of the catheter system 200, energy can be transmitted through the energy guide 222A and can be directed through the guide distal end 222D and toward the plasma generator 233 such that plasma can be generated in the catheter fluid 232 within the balloon interior 246 of the balloon 202. The guide distal end 222D can have any suitable shape such that the energy transmitted through the energy guide 222A can be effectively and accurately directed through the guide distal end 222D and toward the plasma generator 233. In one embodiment, the guide distal end 222D can have a flat, cleaved end through which the energy is directed toward the plasma generator 233. Alternatively, the guide distal end 222D can be generally semi-spherical, ball-shaped, conical, wedge-shaped, pyramidal or can be another suitable shape.

In some embodiments, as shown in Figure 2, the plasma generator 233 (or target) can include a proximal end 233P that is angled or otherwise configured to more effectively direct and/or concentrate the energy in the form of the plasma that has been generated in the catheter fluid 232 through the section opening 272 in the connector section 266 of the emitter housing 260 and toward the balloon wall 230 positioned adjacent to the vascular lesions 106A at the treatment site 106. It is appreciated that the proximal end 233P of the plasma generator 233 can be configured at any suitable angle so as to effectively direct and/or concentrate the plasma energy as desired. For example, in some such embodiments, the proximal end 233P of the plasma generator 233 can be angled at between approximately 5 degrees and 45 degrees relative to a flat, perpendicular configuration. Alternatively, the proximal end 233P of the plasma generator 233 can be angled at less than 5 degrees or greater than 45 degrees relative to a flat, perpendicular configuration in order to direct energy in the form of the plasma that has been generated in the catheter fluid 232 toward the balloon wall 230 positioned adjacent to the treatment site 106.

The plasma generator 233 can be formed from any suitable materials. For example, in certain non-exclusive embodiments, the plasma generator 233 can be formed from one or more metals such as titanium, stainless steel, tungsten, etc. Alternatively, the plasma generator 233 may be formed from plastics such as polyimide and nylon. Still alternatively, the plasma generator 233 can be formed from other suitable materials. It is appreciated that in different embodiments, the plasma generator 233 can be formed from the same materials as the emitter housing 260 or different materials from the emitter housing 260.

It is appreciated that during use of the catheter system 200, the catheter fluid 232 that is utilized to inflate the balloon 204 also is allowed to enter into the area of the connector section 266 of the emitter housing 260 through the section opening 272. Subsequently, the pulsed energy that is directed through the energy guide 222A and toward the plasma generator 233 generates a plasma-induced bubble 134 (illustrated in Figure 1) in the catheter fluid 232 in the general area of the connector section 266 of the emitter housing 260. As the bubble 134 expands, it is directed and/or focused by the proximal end 233P of the plasma generator 233 through the section opening 272 of the connector section 266 and toward the balloon wall 230 positioned adjacent to the vascular lesions 106A at the treatment site 106.

Figure 3 is a simplified schematic perspective view illustration of the emitter assembly 229 illustrated in Figure 2. More particularly, Figure 3 is a simplified schematic perspective view illustration showing a portion of the energy guide 222A, the plasma generator 233, and the emitter housing 260, including the first housing section 262, the second housing section 264, and the connector section 266, that together form the emitter assembly 229.

As shown, the first housing section 262 can be substantially annular-shaped and/or cylindrical-shaped, and can include the small housing gap 368 that that extends fully along a length of the first housing section 262 and that allows for slight expansion or contraction of the first housing section 262 due to changes in environmental conditions in which the catheter system 200 (illustrated in Figure 2) is being used.

Figure 3 also illustrates a first housing coupler 374 that is used for purposes of coupling the first housing section 262 to a portion of the energy guide 222A, such as at or near the guide distal end 222D of the energy guide 222A. The design of the first housing coupler 374 can be varied. For example, in one embodiment, the first housing coupler 374 can include an adhesive material that is positioned between an outer surface of the energy guide 222A and an inner surface of the first housing section 262 in order to effectively couple and/or secure the first housing section 262 to the energy guide 222A. Alternatively, the first housing coupler 374 can have another suitable design.

The type of adhesive materials used with the first housing coupler 374 for securing the first housing section 262 to the energy guide 222A can be varied. For example, in certain embodiments, adhesive materials with low hardness properties (such as silicone-based adhesives) may be chosen to dampen/lessen the shockwave force to the energy guide 222A. Alternatively, other suitable adhesive materials may be chosen.

In some embodiments, an extruded thermal plastic tubing (made from materials such as Pebax^{®}, nylon, polyurethane, etc.) may be used to add a soft protective layer 375 between the energy guide 222A and the first housing section 262 of the emitter housing 260. Such protective layer 375 can also help to center the energy guide 222A in a manner that is slightly offset from the inner diameter of the emitter housing 260 to promote proper alignment with the plasma generator 233.

As illustrated, the second housing section 264 can be substantially annular-shaped and/or cylindrical-shaped, and can include the small housing gap 370 that that extends fully along a length of the second housing section 264 and that allows for slight expansion or contraction of the second housing section 264 due to changes in environmental conditions in which the catheter system 200 is being used.

Figure 3 also illustrates a second housing coupler 376 that is used for purposes of coupling the second housing section 264 to the plasma generator 233. The design of the second housing coupler 376 can be varied. For example, in one embodiment, the second housing coupler 376 can include an adhesive material that is positioned between an outer surface of the plasma generator 233 and an inner surface of the second housing section 264 in order to effectively couple and/or secure the second housing section 264 to the plasma generator 233. It is appreciated that, in such embodiments, any suitable type of adhesive materials can be used for the second housing coupler 376. Alternatively, the second housing coupler 376 can have another suitable design and/or the second housing section 264 can be secured to the plasma generator 233 in another suitable manner. For example, a crimping process can crimp the plasma generator 233 mechanically in place within the second housing section 264. Still alternatively, the plasma generator 233 may also be press fit into the second housing section 264. Yet alternatively, the housing gap 368 may function as an expansion slot which may assist with an interference fit between the second housing section 264 and the plasma generator 233. The housing gap 368 or expansion slot may be made wide enough to minimize the outer diameter of the emitter housing to inner shaft, since it removes the wall thickness of the emitter housing 260 on the top end.

As further illustrated in Figure 3, the generally flat, cleaved end of the guide distal end 222D faces across the connector section 266 of the emitter housing 260 toward the angled proximal end 233P of the plasma generator 233.

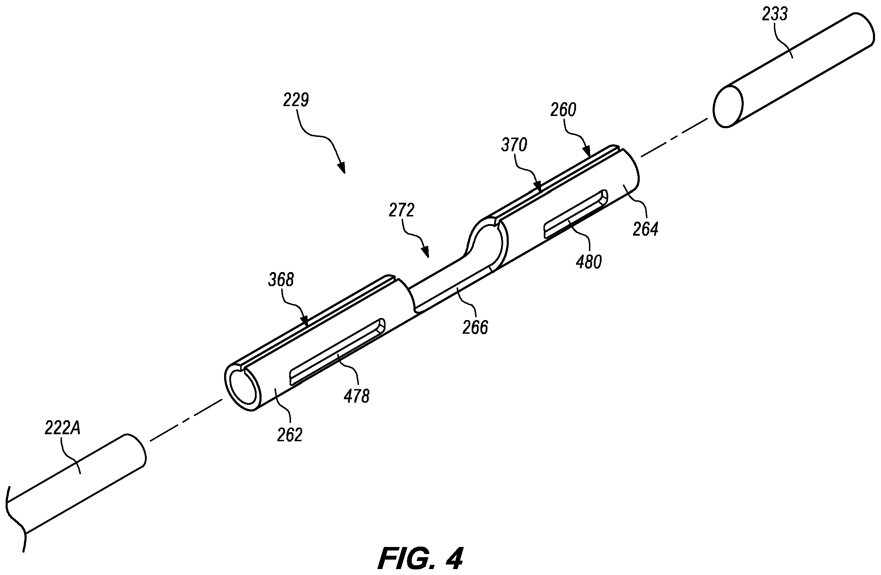

Figure 4 is a simplified schematic exploded view illustration of the emitter assembly 229 illustrated in Figure 2. More particularly, Figure 4 illustrates an exploded view of the emitter assembly 229 including at least a portion of the energy guide 222A, the plasma generator 233, and the emitter housing 260. Figure 4 also illustrates the small housing gaps 368, 370 that can be formed into the first housing section 262 and the second housing section 264, respectively, of the emitter housing 260.

Also shown in Figure 4 are (i) a first housing port 478 that is formed into the first housing section 262 of the emitter housing 260, through which an adhesive material can be introduced in order to effectively secure the first housing section 262 to a portion of the energy guide 222A, such as at or near the guide distal end 222D of the energy guide 222A; and (ii) a second housing port 480 that is formed into the second housing section 264 of the emitter housing 260, through which an adhesive material can be introduced in order to effectively secure the second housing section 264 to the plasma generator 233.

Figure 4 also more clearly illustrates the shape of one embodiment of the connector section 266, including the section opening 272 through which the plasma energy can be directed toward the balloon wall 230 (illustrated in Figure 2) that is positioned adjacent to the vascular lesions 106A (illustrated in Figure 1) at the treatment site 106 (illustrated in Figure 1).

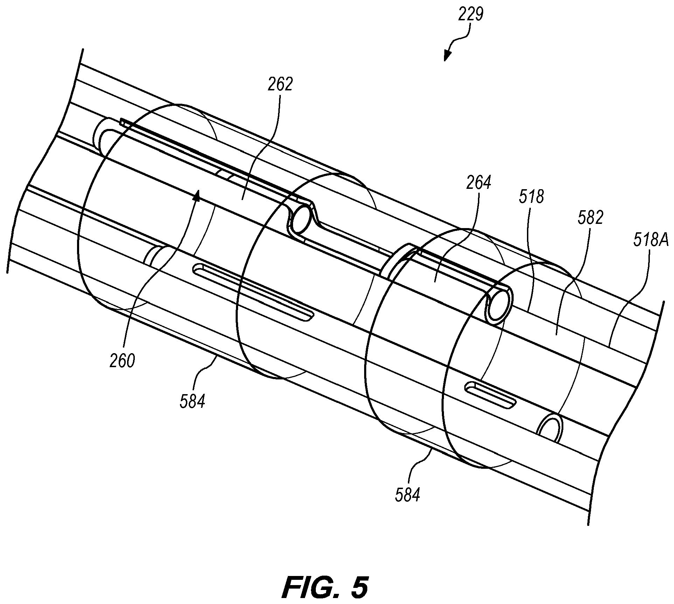

Figure 5 is a simplified schematic perspective view illustration of the emitter assembly 229 illustrated in Figure 2 that is secured to a guidewire lumen 518 of the catheter system 200 (illustrated in Figure 2).

As illustrated in this embodiment, the guidewire lumen 518 can include one or more grooves 582 that are formed along and/or into an outer surface 518A of the guidewire lumen 518. The emitter assembly 229 can then be positioned within one of the grooves 582 and can be held in position within the groove 582 by one or more assembly attachers 584 (two are illustrated in Figure 5). As shown, a first assembly attacher 584 can be positioned substantially adjacent to the first housing section 262 of the emitter housing 260, and a second assembly attacher 584 can be positioned substantially adjacent to the second housing section 264 of the emitter housing 260, in order to effectively hold the emitter assembly 229 in position within the groove 582 formed into the outer surface 518A of the guidewire lumen 518.

The assembly attachers 584 can have any suitable design. In some embodiments, as shown in Figure 5, the assembly attachers 584 can be provided in the form of a heat shrink-style attacher. Alternatively, the assembly attachers 584 can have another suitable design.

It is appreciated that a second emitter assembly 229 is also shown in Figure 5 as being held in position within another one of the grooves 582 formed into the outer surface 518A of the guidewire lumen 518.

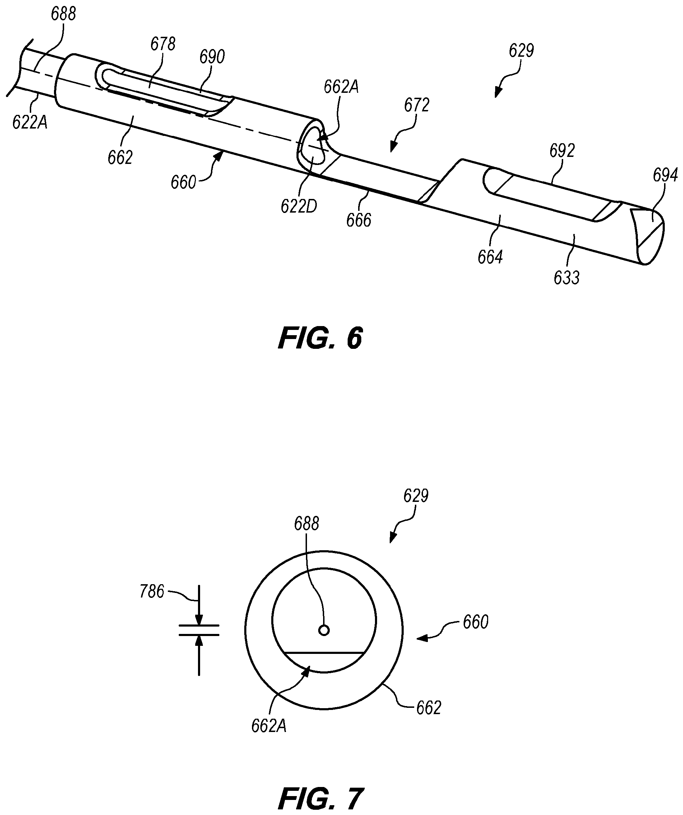

Figure 6 is a simplified schematic perspective view illustration of another embodiment of the emitter assembly 629. As shown in Figure 6, the emitter assembly 629 is somewhat similar in design, positioning and function to the previous embodiments. In this embodiment, the emitter assembly 629 again includes at least part of an energy guide 622A, a plasma generator 633, and an emitter housing 660. The emitter housing 660 again also includes (i) a first housing section 662, including a guide aperture 662A, that is configured to at least substantially encircle a portion of the energy guide 622A, such as at or near a guide distal end 622D of the energy guide 622A; (ii) a second housing section 664; and (iii) a connector section 666, again including a section opening 672, that is coupled to, integrally formed with and/or extends between the first housing section 662 and the second housing section 664. In this embodiment, the emitter assembly 629 is again configured to effectively direct and/or concentrate energy generated in the catheter fluid 232 (illustrated in Figure 2) that is retained within the balloon 204 (illustrated in Figure 2) so as to impart pressure onto and induce fractures in the vascular lesions 106A (illustrated in Figure 1) at the treatment site 106 (illustrated in Figure 1).

However, as shown in the embodiment illustrated in Figure 6, the plasma generator 633 is integrally formed with the second housing section 664 of the emitter housing 660, rather than being positioned and/or secured substantially within the second housing section as in previous embodiments.

With such design, the emitter housing 660 can be fabricated by machining a single piece of rod using instead of making it out of a hypotube, as is typically used for the embodiment of the emitter housing 260 illustrated in Figure 2. Machining the emitter housing 660 can be achieved through the use of any suitable machining processes. For example, in certain non-exclusive implementations, machining of the emitter housing 660 can be achieved by electrical discharge machining, or micro machining using milling or swiss screw machining techniques.

It is appreciated that there may be a few key advantages of this design as compared to a hypotube design. First, this integrated design where the emitter housing 660 is formed from a single piece of rod allows for the guide aperture 662A that is formed into the first housing section 662 and is configured to receive and retain the portion of the energy guide 622A to be offset an offset distance 786 (illustrated in Figure 7) from a central axis 688 of the emitter housing 660, therefore allowing the connector section 666 to be thicker in dimension. Second, this integrated design maximizes the cross-sectional area of the plasma generator 633 since it is made out of the same material as the second housing section 664, whereas in the hypotube design the plasma generator material and hypotube material may be different, therefore the wall of the hypotube reduces the area of the plasma generator cross-section. It is desirable to maximize the cross-section of the plasma generator 633 to reduce the need for precise alignment of the guide distal end 622D of the energy guide 622A relative to the plasma generator 633. Third, as shown, the first housing section 662 and the second housing section 664 can include cut outs 690, 692, respectively, to accommodate the assembly attachers 584 (illustrated in Figure 5), such as heat shrink-style attachers, pieced to the inner member shaft can be made to reduce crossing profile of the balloon catheter assembly. Fourth, in some embodiments, a first housing port 678, such as a glue port to accommodate the addition of adhesive material between the first housing section 662 and the energy guide 622A, can be combined with the cut out 690 in the first housing section 662 to allow adhesive to wick inside for consistent bonding. Fifth, radii 694 can be cut into the emitter housing 660 to reduce the number of sharp edges on the emitter housing 660, to inhibit potential damage to the balloon 204. Lastly, compared to the hypotube design, there is no need to bond a plasma generator into the emitter housing 660 since the plasma generator 633 is already integrated within the second housing section 664 of the emitter housing 660.

Figure 7 is a simplified schematic end view illustration of a portion of the emitter assembly 629 illustrated in Figure 6. In particular, Figure 7 is a simplified end view looking directly at the first housing section 662 of the emitter housing 660, which shows the guide aperture 662A that has been formed into the emitter housing 660 for purposes of receiving and retaining the portion of the energy guide 622A (illustrated in Figure 6). As illustrated, the guide aperture 662A is offset an offset distance 786 from the central axis 688 (illustrated as a small circle) of the emitter housing 660, therefore allowing the connector section 666 (illustrated in Figure 6) to be thicker in dimension. In certain non-exclusive embodiments, the guide aperture 662A can be offset from the central axis 688 of the emitter housing 660 by an offset distance 786 of between approximately 0.010 inches and 0.020 inches. In one such embodiment, the guide aperture 662A can be offset from the central axis 688 of the emitter housing 660 by an offset distance 786 of approximately 0.015 inches. Alternatively, the guide aperture 662A can be offset from the central axis 688 of the emitter housing 660 by an offset distance 786 of greater than 0.020 inches or less than 0.010 inches.

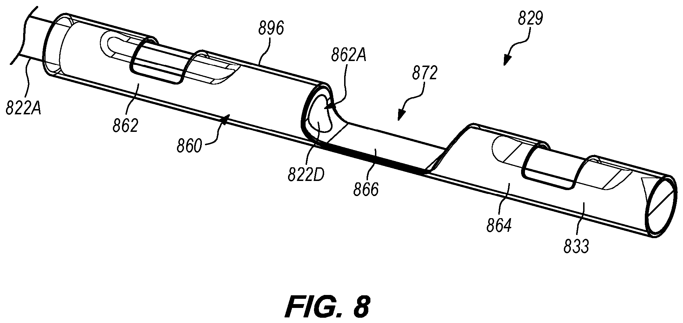

Figure 8 is a simplified schematic perspective view illustration of still another embodiment of the emitter assembly 829. In this embodiment, the emitter assembly 829 is again configured to effectively direct and/or concentrate energy generated in the catheter fluid 232 (illustrated in Figure 2) that is retained within the balloon 204 (illustrated in Figure 2) so as to impart pressure onto and induce fractures in the vascular lesions 106A (illustrated in Figure 1) at the treatment site 106 (illustrated in Figure 1).

As shown in Figure 8, the emitter assembly 829 is somewhat similar in design, positioning and function to the embodiment illustrated in Figure 6, and thus is able to realize most, if not all, of the same advantages noted above. For example, in this embodiment, the emitter assembly 829 again includes at least part of an energy guide 822A, a plasma generator 833, and an emitter housing 860. The emitter housing 860 again also includes (i) a first housing section 862, including a guide aperture 862A, that is configured to at least substantially encircle a portion of the energy guide 822A, such as at or near a guide distal end 822D of the energy guide 822A; (ii) a second housing section 864 that is integrally formed with the plasma generator 833; and (iii) a connector section 866, again including a section opening 872, that is coupled to, integrally formed with and/or extends between the first housing section 862 and the second housing section 864.

With such design, the emitter housing 860 can again be fabricated by machining a single piece of rod using instead of making it out of a hypotube, as is typically used for the embodiment of the emitter housing 260 illustrated in Figure 2. Machining the emitter housing 860 can be achieved through the use of any suitable machining processes. For example, in certain non-exclusive implementations, machining of the emitter housing 860 can be achieved by electrical discharge machining, or micro machining using milling or swiss screw machining techniques.

However, as shown in the embodiment illustrated in Figure 8, the emitter assembly 829 further includes a reinforcement cover 896 that is positioned about, placed over and/or substantially encircles the emitter housing 860. In some embodiments, the reinforcement cover 896 can be provided in the form of a polyimide tube, which, as shown, can be notched to match the design of the emitter housing 860, and is positioned about, placed over and/or substantially encircles the emitter housing 860 and bonded in place, such as with a UV cured adhesive. Alternatively, the reinforcement cover 896 can be formed from other materials and/or have another suitable design.

The reinforcement cover 896 serves to reinforce the structure of the solid emitter housing 860 and/or plasma generator 833 against the repetitive forces exerted during normal device function (acoustic pressure forces generated by the plasma initiation). Furthermore, in the event the solid emitter housing 860 and/or plasma generator 833 does become damaged, the reinforcement cover 896 can be further configured to contain any pieces or fragments that may be generated, thereby preventing them from becoming completely separated from the emitter housing 860.

As with previous embodiments, the emitter housing 860 and/or the plasma generator 833 can be formed from any suitable materials. For example, in some non-exclusive embodiments, the emitter housing 860 and/or the plasma generator 833 can be formed from one or more of metals such as titanium, stainless steel, tungsten, etc. Alternatively, the emitter housing 860 and/or the plasma generator 833 can be formed from other suitable materials.

It is further appreciated that, in certain non-exclusive alternative embodiments, the reinforcement cover 896 can also be utilized with one or more of the other embodiments of the emitter assembly illustrated and described in detail herein.

In various embodiments, the catheter systems and related methods disclosed herein can include a catheter configured to advance to a vascular lesion, such as a calcified vascular lesion or a fibrous vascular lesion, at a treatment site located within or adjacent a blood vessel or a heart valve within a body of a patient. The catheter includes a catheter shaft, and an inflatable balloon that is coupled and/or secured to the catheter shaft. The balloon can include a balloon wall that defines a balloon interior. The balloon can be configured to receive a catheter fluid within the balloon interior to expand from a deflated state suitable for advancing the catheter through a patient's vasculature, to an inflated state suitable for anchoring the catheter in position relative to the treatment site. The present technology is also directed toward methods for treating a treatment site within or adjacent to a vessel wall, with such methods utilizing the devices disclosed herein.

It should be noted that, as used in this specification and the appended claims, the singular forms "a," "an," and "the" include plural referents unless the content and/or context clearly dictates otherwise. It should also be noted that the term "or" is generally employed in its sense including "and/or" unless the context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

It is recognized that the figures shown and described are not necessarily drawn to scale, and that they are provided for ease of reference and understanding, and for relative positioning of the structures.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions and sub-combinations thereof. It is therefore intended that the following appended claims and claims hereafter introduced are interpreted to include all such modifications, permutations, additions and sub-combinations as are within their true spirit and scope, and no limitations are intended to the details of construction or design herein shown.
The following aspects are preferred embodiments of the present invention.
1. A catheter system for treating a treatment site within or adjacent to a blood vessel or a heard valve within a body of a patient the catheter system comprising:
   an energy source that generates energy;
   a catheter fluid, and
   an emitter assembly including (i) at least a portion of an energy guide having a guide distal and that is selectively positioned near the treatment site, (ii) a plasma generator, and (iii) an emitter housing that is secured to each of the energy guide and the plasma generator to mainiain a relative position between the guide distal end of the energy guide and the plasma generator, the energy guide being configured to receive energy from the energy source and direct the energy loward the plasma generator to generate a plasma bubble in the catheter fluid, and the plasma generator being configured to direct energy from the plasma bubble toward the treatment site.
2. The catheter system of aspect 1 wherein the emitter housing includes (i) a first housing section that is secured to the energy guide at or near the guide distal end, (ii) a second housing section that is one of secured to and integrally formed with the plasma generator, and (iii) a connector section that is coupled to and extends between the first housing section and the second housing section.
3. The catheter system of aspect 2 wherein the first housing section is substantially cylindrical-shaped.
4. The catheter system of aspect 3 wherein the first housing section includes a housing gap that extends substantially along a length of the first housing section, the first housing section being configured to expand and contract due to changes in environmental conditions
5. The catheter system of any of aspects 2-4 wherein the first housing section includes a guide aperture, at least a portion of the energy guide being secured within the guide aperture.
6. The catheter system of any of aspects 2-5 further comprising adhesive material that secures the first housing section to the energy guide at or near the guide distal end.
7. The catheter system of aspect 6 wherein the first housing section includes a first housing port positioned between the first housing section and the energy guide, the first housing port being configured to receive the adhesive material so that the adhesive material secures the first housing section to the energy guide at or near the guide distal end.
8. The catheter system of any one of aspects 2-7 wherein the second housing section is substantially cylindrical-shaped.
9. The catheter system of aspect 2 wherein the second housing section includes a housing gap that extends substantially along a length of the second housing section, the second housing section being configured to expand and contract due to changes in environmental conditions.
10. The catheter system of any of aspects 2-9 wherein the second housing section includes a generator aperture, at least a portion of the plasma generator being secured within the generator aperture.
11. The catheter system of any one of aspects 6-7 wherein the adhesive material secures the second housing section to the plasma generator.
12. The catheter system of aspect 11 wherein the second housing section includes a second housing port positioned between the second housing section and the energy guide, the second housing port being configured to receive the adhesive material so that the adhesive material secures the second housing section to the energy guide at or near the guide distal end.
13. The catheter system of any one of aspects 2-12 wherein the second housing section is integrally formed with the plasma generator.
14. The catheter system of any one of aspects 2-13 wherein the connector section includes a section opening, the plasma generator being configured to direct the energy from the plasma bubble through the section opening and toward the treatment site.
15. The catheter system of aspect 14 wherein the connector section is partially cylindrical-shaped, the section opening extending substantially along a length of the connector section.
16. The catheter system of any one of aspects 14-15 wherein the plasma generator has a proximal end that is angled so that the plasma generator is configured to direct the energy from the plasma bubble through the section opening and toward the treatment site.
17. The catheter system of aspect 16 wherein the proximal end of the plasma generator is angled at between approximately 5 degrees and 45 degrees relative to a flat, perpendicular configuration.
18. The catheter system of any one of aspects 1-17 further comprising a reinforcement cover that is positioned to substantially encircle the emitter housing.
19. The catheter system of aspect 18 wherein the reinforcement cover includes a polyimide tube.
20. The catheter system of any one of aspects 1-19 further comprising a guidewire lumen that includes an outer surface having a groove, the emitter housing being positioned within the groove.
21. The catheter system of aspect 20 further comprising a first assembly attacher that is positioned adjacent to the first housing section, and a second assembly attacher that is positioned adjacent to the second housing section, the assembly attachers being configured to retain the emitter housing within the groove formed along the outer surface of the guidewire lumen.
22. The catheter system of any one of aspects 1-21 further comprising a balloon including a balloon wall that defines a balloon interior, the balloon being configured to retain the catheter fluid within the balloon interior; and wherein the guide distal end, the plasma generator and the emitter housing are positioned within the balloon interior.
23. The catheter system of aspect 22 wherein the balloon is selectively inflatable with the catheter fluid to expand to an inflated state, wherein when the balloon is in the inflated state the balloon wall is configured to be positioned substantially adjacent to the treatment site.
24. The catheter system of aspect 23 wherein the plasma generator is configured to direct the energy from the plasma bubble toward a portion of the balloon wall that is positioned substantially adjacent to the treatment site.
25. The catheter system of aspect 1 wherein the energy guide generates one or more pressure waves in the catheter fluid that impart a force upon the treatment site.
26. The catheter system of aspect1 wherein the energy guide includes an optical fiber.
27. The catheter system of aspect 1 wherein the energy source includes a laser.
28. The catheter system of aspect 1 wherein the catheter fluid includes one of a wetting agent and a surfactant.
29. A method for treating a treatment site within or adjacent to a blood vessel or a heart valve within a body of a patient that utilizes the catheter system of any of aspects 1-28.
30. A method for treating a treatment site within or adjacent to a blood vessel or a heart valve within a body of a patient, the method comprising the steps of:
   generating energy with an energy source;
   positioning an emitter assembly within a catheter fluid near the treatment site, the emitter assembly including (i) at least a portion of an energy guide having a guide distal end that is selectively positioned near the treatment site, (ii) a plasma generator, and (iii) an emitter housing that is secured to each of the energy guide and the plasma generator to maintain a relative position between the guide distal end of the energy guide and the plasma generator;
   receiving energy from the energy source with the energy guide;
   generating a plasma bubble in the catheter fluid with the energy from the energy guide that is directed toward the plasma generator; and
   directing energy from the plasma bubble with the plasma generator toward the treatment site.

## Claims

1. An emitter assembly (129, 229, 629, 829) for a catheter system (100, 200) for treating a treatment site (106, 106A) within or adjacent to a blood vessel (108) or a heart valve within a body (107) of a patient (109), the emitter assembly (129, 229, 629, 829) comprising:
(i) at least a portion of an energy guide (122A, 222A, 622A, 822A) having a guide distal end that is selectively positioned near the treatment site (106, 106A),
(ii) a plasma generator (133, 233, 633, 833), and
(iii) an emitter housing (260, 660, 860) that is secured to each of the energy guide (122A, 222A, 622A, 822A) and the plasma generator (133, 233, 633, 833) to maintain a relative position between the guide distal end of the energy guide (122A, 222A, 622A, 822A) and the plasma generator (133, 233, 633, 833), the energy guide (122A, 222A, 622A, 822A) being configured to receive energy from an energy source (124) and direct the energy toward the plasma generator (133, 233, 633, 833) to generate a plasma bubble (134) in a catheter fluid (132, 232), and the plasma generator (133, 233, 633, 833) being configured to direct energy from the plasma bubble (134) toward the treatment site (106, 106A)
wherein the emitter housing (260, 660, 860) includes a first housing section (262, 662, 862) that is secured to the energy guide (122A, 222A, 622A, 822A) at or near the guide distal end, and
wherein the first housing section (262, 662, 862) includes a guide aperture (362A, 662A, 862A), at least a portion of the energy guide (122A, 222A, 622A, 822A) being secured within the guide aperture (362A, 662A, 862A).

2. The emitter assembly (129, 229, 629, 829) of claim 1 wherein the first housing section (262, 662, 862) includes a cut out (690), preferably wherein the cut out (690) is configured for accommodating an assembly attacher (584), such as a first assembly attacher (584).

3. The emitter assembly (129, 229, 629, 829) of claims 1 or 2, wherein the emitter housing includes a second housing section (264, 664, 864) that is one of secured to and integrally formed with the plasma generator (133, 233, 633, 833), and a connector section (266, 666, 866) that is coupled to and extends between the first housing section (262, 662, 862) and the second housing section (264, 664, 864).

4. The emitter assembly (129, 229, 629, 829) of claim 3 wherein the second housing section (264, 664, 864) includes a cut out (692), preferably wherein the cut out (692) is configured for accommodating another assembly attacher (584), such as a second assembly attacher (584).

5. The emitter assembly (129, 229, 629, 829) of any one of the preceding claims, wherein the guide aperture (662A) is configured to offset the portion of the energy guide (622A) an offset distance (786) from a central axis (688) of the emitter housing (660).

6. The emitter assembly (129, 229, 629, 829) of any one of the preceding claims wherein the first housing section (262, 662, 862) is substantially cylindrical-shaped, and wherein the first housing section (262, 662, 862) includes a housing gap (368, 370) that extends substantially along a length of the first housing section (262, 662, 862), the first housing section (262, 662, 862) being configured to expand and contract due to changes in environmental conditions.

7. The emitter assembly (129, 229, 629, 829) of any one of the preceding claims wherein the first housing section (262, 662, 862) includes a housing port (478, 678) positioned between the first housing section (262, 662, 862) and the energy guide (122A, 222A, 622A, 822A), the housing port (478, 678) being configured to receive an adhesive material so that the adhesive material secures the first housing section (262, 662, 862) to the energy guide (122A, 222A, 622A, 822A) at or near the guide distal end.

8. The emitter assembly (129, 229, 629, 829) of claim 7 in combination with claim 4, wherein the cut out (690) is configured to allow the adhesive material to wick inside the first housing section (262, 662, 862) for bonding.

9. The emitter assembly (129, 229, 629, 829) of any one of claims 3-8 wherein the second housing section (264, 664, 864) is integrally formed with the plasma generator (133, 233, 633, 833).

10. The emitter assembly (129, 229, 629, 829) of any one of claims 3-9 wherein the connector section (266, 666, 866) is partially cylindrical-shaped and includes a section opening (272, 672, 872) that extends substantially along a length of the connector section (266, 666, 866), the plasma generator (133, 233, 633, 833) being configured to direct the energy from the plasma bubble (134) through the section opening (272, 672, 872) and toward the treatment site (106, 106A).

11. The emitter assembly (129, 229, 629, 829) of claim 10 wherein the plasma generator (133, 233, 633, 833) has a proximal end (104P, 122P, 204P, 233P) that is angled so that the plasma generator (133, 233, 633, 833) is configured to direct the energy from the plasma bubble (134) through the section opening (272, 672, 872) and toward the treatment site (106, 106A).

12. The emitter assembly (129, 229, 629, 829) of any one of the preceding claims further comprising a guidewire lumen (118, 518) that includes an outer surface (518A) having a groove (582), the emitter housing (260, 660, 860) being positioned within the groove (582).

13. The emitter assembly (129, 229, 629, 829) of claim 12 further comprising a first assembly attacher that is positioned adjacent to the first housing section (262, 662, 862), and a second assembly attacher that is positioned adjacent to the second housing section (264, 664, 864), the assembly attachers (584) being configured to retain the emitter housing (260, 660, 860) within the groove (582) formed along the outer surface (518A) of the guidewire lumen (118, 518).

14. A catheter system (100, 200) for treating a treatment site (106, 106A) within or adjacent to a blood vessel (108) or a heart valve within a body (107) of a patient (109), the catheter system (100, 200) comprising:
- a catheter (102); and
- an emitter assembly (129, 229, 629, 829) according to any one of the preceding claims.

15. The catheter system (100; 200) of claim 14, further comprising:
- an energy source (124), such as a laser, that generates energy; and
- a catheter fluid (132, 232);
wherein the energy guide (122A, 222A, 622A, 822A) is configured to receive the energy from the energy source (124) and direct the energy toward the plasma generator (133, 233, 633, 833) to generate the plasma bubble (134) in the catheter fluid (132, 232), preferably wherein the catheter system (100, 200) further comprises
- a balloon (104, 202, 204) including a balloon wall (130, 230) that defines a balloon interior (146, 246), the balloon (104, 202, 204) being configured to retain the catheter fluid (132, 232) within the balloon interior (146, 246); wherein
- the guide distal end, the plasma generator (133, 233, 633, 833) and the emitter housing (260, 660, 860) are positioned within the balloon interior (146, 246);
- the balloon (104, 202, 204) is selectively inflatable with the catheter fluid (132, 232) to expand to an inflated state, wherein when the balloon (104, 202, 204) is in the inflated state the balloon wall (130, 230) is configured to be positioned substantially adjacent to the treatment site (106, 106A); and
- the plasma generator (133, 233, 633, 833) is configured to direct the energy from the plasma bubble (134) toward a portion of the balloon wall (130, 230) that is positioned substantially adjacent to the treatment site (106, 106A), preferably wherein
- the emitter housing (660) includes one or more radii (694) for reducing a number of sharp edges on the emitter housing (660) for inhibiting potential damage to the balloon (104, 202, 204), and/or
- the energy guide (122A, 222A, 622A, 822A) is configured for generating one or more pressure waves in the catheter fluid (132, 232) for imparting a force upon the treatment site (106, 106A).
